# EUROPEAN PATENT APPLICATION

(11) **EP 4 036 926 A1**
(43) Date of publication of application: **03.08.2022**
(21) Application number: 21154490.3
(22) Date of filing: 01.02.2021
(51) Int. Cl.: G16H 20/40, G16H 40/63, G16H 50/20, A61B 5/00, A61M 16/00, G16H 30/40

(54) **MEDICAL SYSTEM FOR VENTILATION AND IMAGING**

(71) Applicant: Koninklijke Philips N.V., 5656 AG Eindhoven (NL)
(72) Inventor: MAACK, Hanns-Ingo, 5656 AE Eindhoven (NL); WIEBERNEIT, Nataly, 5656 AE Eindhoven (NL); KOEHLER, Thomas, 5656 AE Eindhoven (NL); VON BERG, Jens, 5656 AE Eindhoven (NL)
(74) Representative: Philips Intellectual Property & Standards

(57) **Abstract**

The present disclosure provides a medical system (100) comprising at least one data processing unit (111, 121, 130), a mechanical ventilator (110), configured to be operated to cause a ventilation status at a to-be-ventilated subject, and a medical imaging device (120), configured as a mobile device. The data processing unit (111, 121, 130) is configured to obtain, from the mechanical ventilator (110), ventilation status information associated with the ventilation status of the to-be-ventilated subject. Further, the data processing unit (111, 121, 130) is further configured to trigger acquisition of a first image if the ventilation status meets a first image acquisition criterion. Furthermore, the data processing unit (111, 121, 130) is further configured to trigger acquisition of at least one subsequent, further image if the ventilation status meets a second image acquisition criterion.

## Description

### FIELD OF THE INVENTION

The present invention relates to a medical system, and in particular to a medical system for ventilation and imaging.

### BACKGROUND OF THE INVENTION

There are conditions that may require a subject, e.g. a patient, to be ventilated by using a mechanical ventilator. For example, if the subject has a lung disease, such as chronic obstructive pulmonary disease (COPD), SARS-CoV-2, etc., supportive or external ventilation may be helpful or necessary. At the same time, for diagnosis, monitoring, etc., it may also be helpful if medical imaging is performed on the subject; for example, if the subject has a lung disease that requires ventilation, it may be helpful if imaging of the chest or lungs is performed. When the subject is ventilated, the subject's organs and tissues involved in breathing move, which can affect image acquisition, e.g. in terms of image quality. For example, this can lead to artifacts and/or the image not showing the desired area of interest or not showing the desired quality. In addition, there is no machine-based mutual exchange of information between ventilation and imaging, so medical staff responsible for ventilation and imaging, respectively, may lose information to improve treatment of the subject.

### SUMMARY OF THE INVENTION

There may, therefore, be a need for improved means for a subject being ventilated to be examined. The object of the present invention is solved by the subject-matter of the independent claims, wherein further embodiments are incorporated in the dependent claims.

According to a first aspect, there is provided a medical system, preferably a medical system for ventilation and imaging. The system comprises at least one data processing unit. Further, the system comprises a medical imaging device, configured as a mobile device, and a mechanical ventilator, configured to be operated to cause a ventilation status of a ventilated subject. The system, preferably the data processing unit, is configured to obtain, from the mechanical ventilator, ventilation status information associated with the ventilation status . Further, the system, preferably the data processing unit, is further configured to trigger acquisition of a first image if the ventilation status meets a first image acquisition criterion, and is further configured to trigger acquisition of at least one subsequent, further image if the ventilation status meets a second image acquisition criterion.

In this way, the mechanical ventilator and the medical imaging device work together, for example, to functionally interact and/or exchange data with each other. For example, medical images can always be acquired at the desired moment of ventilation, namely when the respective image acquisition criterion is at least substantially fulfilled. This allows to achieve reproducible medical images, allowing better recognition of change in e.g. a subject's respiratory status. Further, image acquisitions can be triggered in different respiratory phases. However, several image acquisitions may also be triggered in a specific respiratory phase, i.e. in the same respiratory phase in each case. It is also possible to trigger multiple image acquisitions, each with the identical or essentially identical ventilation status, to better compare image acquisitions to each other. It is also conceivable that both the ventilation status and the respiratory phase are used as criteria for triggering the image acquisition, which makes it even easier to compare the image acquisitions with each other. In addition, combining information obtained from the mechanical ventilator and the medical imaging device allows a better assessment of the overall condition of the subject.

Optionally, the medical system may be configured, e.g. via one or more data interfaces, to exchange data between the data processing unit, the mechanical ventilator and/or the medical imaging device.

As used herein, the mechanical ventilator may be configured to provide invasive or noninvasive ventilation to the subject, i.e. a patient, and may comprise one or more of a respiratory unit, such as pump, a patient interface, a controller or data processing unit, a data and/or communication interface, etc.

Further, as used herein, the "ventilation status" may be indicated by one or more of a set of ventilation parameters used to operate the mechanical ventilator to cause the desired ventilation status, and/or ventilation parameters associated with a tidal volume, a minute volume in volume modalities, a peak pressure (in pressure modalities), a respiratory frequency, a positive end expiratory pressure, an inspiratory time, an inspiratory flow, an inspiratory-to-expiratory ratio, a time of pause, a trigger sensitivity, a support pressure, and an expiratory trigger sensitivity, etc. The above ventilation parameters may also be referred to as respiratory parameters. A change in these parameters causes a change in the subject's ventilation and/or respiration, i.e. a change of the ventilation status. The controller of the mechanical ventilator and/or the medical system may be configured to control and/or monitor the ventilation status. Likewise, a controller of the medical imaging device may be configured to at least indirectly control, e.g. via the medical system and/or the mechanical ventilator, or at least obtain the ventilation status, i.e. a ventilation status information, from the mechanical ventilator. It is noted that the ventilation status information may comprise the set of ventilation parameter associated with the ventilation status. The ventilation status may be provided by the mechanical ventilator as a dataset to be computationally processed or read-out.

As used herein, the medical imaging device may be, for example, an ultrasound device, a X-ray device, etc., configured to acquire images of an area of interest, for example, a body part of the subject, such as the chest and/or lungs. It may comprise one or more of a controller or data processing unit, a radiation source, a detector, a data and/or communication interface, etc.

Further, as used herein, the "data processing unit" may comprise any type of data processor, and may form at least a part of and/or may be part of control electronics. The data processing unit may be a single, optionally dedicated, entity that is higher-level than, for example, the mechanical ventilator and the medical imaging device or control electronics thereof. Or, the data processing entity may comprise one or more data processing entities that are part of the mechanical ventilator and/or the medical imaging device.

As used herein, "image acquisition criterion" may comprise a ventilation or breathing phase, e.g. inspiration, maximum inspiration, expiration, maximum expiration etc. For example, it may be specified that image acquisition is only performed when a certain phase is reached or during a certain phase. Further, the criterion may comprise the presence or determination of a certain ventilation status of the subject is to be ventilated or is being ventilated. Further, the criterion may comprise other conditions affecting and/or facilitating medical imaging, such as movement or non-movement of the area of interest to be captured in the image acquisition, or the like.

It is noted that the ventilation status caused, e.g. by using a specific set of ventilation parameters, at least correlate with the settings of the mechanical ventilator and may therefore be referred to as "ventilation settings".

According to an embodiment, the first and second image acquisition criterion may be the same or deviate from each other at most within a tolerance window, e.g. ±1%, ±2%, ±3, ... ±10%, or the like. In other words, the image acquisitions may always be performed with the same or recurring ventilation parameters, i.e., the first image acquisition with ventilation parameters X and the subsequent image acquisition(s) with the same ventilation parameters X. Optionally, the medical system, e.g. the data processing unit etc., may be configured to derive the current ventilation status and/or the current ventilation parameters from the mechanical ventilator, so that the actual ventilation status and/or the actual ventilation parameters may be determined. Further optionally, the medical system, e.g. the data processing unit etc., may be configured to determine the target ventilation parameters, for example the ventilation parameters at the time of the first image acquisition. For example, this allows to achieve reproducible images enabling better recognition of change in e.g. the subject's respiratory status and/or disease progression. Alternatively, given monitoring data over time, e.g. the mechanical ventilation status and/or ventilation parameters over time, a ventilation parameter set may be determined that now best reproduces the patient conditions in the previous acquisition according to its changed conditions. These settings may be different to that of the previous acquisition, but more suitable for comparing both images according to the changed patient status.

In an embodiment, the medical system, e.g. the data processing unit, may further be configured to control adjustment of the set of ventilation parameters on the mechanical ventilator to be operated with if the medical system, e.g. the data processing unit, determines that one or more of the ventilation parameters have changed compared to the acquisition of the first image. In other words, the system may be configured to (re-)establish the first image acquisition criterion if it is not (yet) satisfied for the further image acquisition. For example, the set of ventilation parameters may be stored as data in a machine-readable memory that can be accessed to derive the corresponding data Further, by way of example, the medical system may be configured to generate one or more control signals to be sent to the mechanical ventilator, and optionally also to the medical imaging device, to adjust one or more ventilation parameters, and optionally to control, e.g. trigger, suspend, etc., image acquisition. This allows the image acquisitions to be performed under the same or at least essentially the same conditions in terms of ventilation. It may allow better comparability of the image acquisitions.

According to an embodiment, the first and second image acquisition criterion may differ from each other. In other words, the first image acquisition is performed under a first image acquisition criterion, while the second image acquisition is performed under a second image acquisition criterion different therefrom. For example, one of the first and second images may be acquired at the moment of maximum inspiration of the subject. This moment may, for example, be determined based on the ventilation parameters, data from a subject monitoring system, or the like. Further, by way of example, the other one of the first and second images may be acquired at the moment of maximum expiration, meaning with a minimum volume of air in the lungs of the subject. Each of the image acquisitions made at these moments allows further diagnostics to be made. By way of example, a lung volume may be determined from the image at maximum full inspiration, which measured volume may be referred to as "total lung capacity (TLC)". Further, for example, a lung volume may be determined from the image at maximum full expiration, which measured volume may be referred to as "residual volume (RV)".

For example, TLC and/or RV may be determined by the following method. First, e.g. a 2D X-ray image of the subject's chest may be provided. The image may be segmented to identify lung structures to provide segmented image data separated from un-segmented areas. Then, spatial lung volume information may be extracted from the image data using the segmented image data derived from the image data. Optionally, by using the extracted spatial lung volume information, a lungs symmetry information using the extracted spatial lung may be determined. In other words, a lung volume may be approximated from a 2D X-ray image of a patient's chest. This is possible because for a respective area of the lungs structure in the X-ray image, the intensity of the image at that area is related to the amount of air present in the lung structure at that area on the image. Therefore, for each respective area over the lung structure, a summation of the pixel intensity values corresponding to that area can allow an approximation of the lungs volume to be found for that area. According to an exemplary embodiment, image data of the first and/or further image may also comprise pixel area information, in other words, the pixel pitch of the detector in e.g. mm², cm², etc. The advantage of this is that after segmentation, the area of each lung in mm² can be calculated. The darkness or brightness of the lungs is a measure of a transition passage filled with air, so it can be re-mapped to the area of the lung in millimeters or centimeters squared. Integrating the pixel area over the lung structure leads to a figure, which is closely correlated to the volume of the lungs. In this way, TLC and RV may provide a reproducible objective figure of merit for the purpose of identifying lung conditions, wherein these may be compared to the ventilation status, i.e. the current ventilation state, and/or the ventilation parameters used for ventilation of the subject.

In an embodiment, at least one of the first and second image acquisition criterion may be maximum inspiration of a ventilated subject. This may be understood as a maximum volume of air in the lungs during, e.g. a normal or typical, breathing cycle. The respective moment of maximum inspiration may, for example, be determined based on the ventilation status, the ventilation parameters, data from a subject monitoring system, or the like. Accordingly, the respective image acquired at maximum inspiration may be referred to as "inspiration image".

In an embodiment, at least one of the first and second image acquisition criterion may be maximum full inspiration of a ventilated subject. This may be understood as a maximum "possible" or "achievable" volume of air in the lungs. The respective moment of maximum full inspiration may, for example, be determined based on the ventilation status, the ventilation parameters, data from a subject monitoring system, or the like. Note that this image is closer to a standard diagnostic chest x-ray image where the patient is asked to perform the breathing maneuver of full inspiration. Accordingly, the respective image acquired at maximum inspiration may be referred to as "full inspiration image".

According to an embodiment, at least one of the first and second image acquisition criterion may be maximum (full) expiration of a ventilated subject. Accordingly, the respective image acquired at maximum (full) expiration may be referred to as "expiration image" or "full expiration image".

For example, to trigger the expiration image, the ventilation status, the ventilation parameters, etc. may be evaluated and/or a measurement of the breathing activity of the subject may be performed. Alternatively, a number of X-ray frames may be acquired, for example, with 5, 10, 15, 20 fps, and monitor the lung volume. Optionally, image acquisition may be terminated if the minimum volume is surpassed. This procedure may also apply to triggering the (full) inspiration image.

In an embodiment, the medical system, e.g. the data processing unit, may further be configured to generate another image, which may be referred to as a "ventilation image", by displaying the one of the first and second image acquired at maximum (full) expiration based on a histogram of the other one of the first and second image acquired at maximum (full) inspiration. In other words, the (full) expiration image may be displayed based on the histogram of the (full) inspiration image. This means that the (full) expiration image is not displayed based on its own histogram, but on that of the (full) inspiration image. For this purpose, the image(s) may be rendered as appropriate. In this way, the (full) expiration image is brighter than the (full) inspiration image at positions, areas, etc., where there is less air in the lungs. The positions, areas, etc. where there is less air in the lungs means good ventilation. In contrast, darker positions, areas, etc. within the (full) expiration image indicate little or less air exchange. This allows lung sections of good ventilation to be distinguished from lung sections of less good ventilation.

According to an embodiment, the medical system, e.g. the data processing unit, is further configured to determine one or more positions and/or areas where the image acquired at maximum (full) expiration has higher brightness than indicated by the histogram of the image acquired at maximum (full) inspiration. For example, the ventilation image may be divided, e.g. segmented, into sections of different brightness by means of image processing. Optionally, sections of different brightness may be highlighted by using different colors, for example green color for sections indicating less air in the lungs and red color for sections indicating little or less air exchange of the lungs. This ventilation image may be minimized in size as a "stamp image", as the main information is the color that does not require good spatial resolution. For example, it may have a matrix of dozens or a few hundred pixels, e.g. 100 x 100 pixels.

In an embodiment, the medical system, e.g. the data processing unit, may be further configured to control displaying the ventilation image at the mechanical ventilator. For example, image data comprising the ventilation image may be transmitted, via one or more data interfaces, from the medical imaging device or another place of data processing to the mechanical ventilator to be displayed. Optionally, the ventilation image may be updated regularly. In this way, medical staff, such as a physician, can monitor the change of the ventilation over time and/or compare it with the applied ventilation status, the ventilation parameters, etc.. Thus, the ventilation image can be considered a feedback on the applied ventilation.

According to an embodiment, the medical system, e.g. the data processing unit, may be further configured to generate a respiratory image by subtracting the image acquired at minimum inspiration or maximum expiration and the image acquired at maximum inspiration from each other. The respiratory image shows regional ventilation or spatial vital capacity volume. This allows the ventilation to be monitored in greater detail.

In an embodiment, the medical system, e.g. the data processing unit, may further be configured to determine tidal volume information from the one of the first and further image, by image acquisition at maximum inspiration and maximum expiration. The or spatial vital capacity volume and/or a tidal volume is a relevant parameter for ventilation. The tidal volume can be adjusted at the mechanical ventilator. By providing the spatial vital capacity volume and/or tidal volume based on the acquired images, the ventilation status caused and/or the ventilation parameters used can be verified, adjusted, or evaluated in the images acquired. In addition, the tidal volume indicates areas of poor ventilation and may therefore help to adjust one or more of the ventilation parameters.

According to an embodiment, the medical system, e.g. the data processing unit, may further be configured to include ventilation information data comprising or derived from the set of ventilation parameters used to acquire the first and/or further image into image data assigned to the first and/or further image. For example, Intensive Care Unit (IC) subjects regularly undergo chest medical imaging to monitor e.g. disease progression and to check for the onset of conditions such as pneumothorax. Commonly, it is desired to evaluate if the image from one day looks very similar to the one from the previous day. However, if a subject is ventilated, the ventilation parameters need to be adjusted from time to time. By including the ventilation information data into the image data, e.g. in an image header, DCOM data, or the like, these data may be provided together to facilitate evaluation of the ventilation and/or subject's condition.

In an embodiment, the medical system, e.g. the data processing unit, may further be configured to control display of ventilation information data comprising or derived from the set of ventilation parameters used to acquire the first and/or further image in one view together with the first and/or further image. In this way, evaluation of the ventilation and/or subject's condition may be improved.

According to an embodiment, the medical system, e.g. the data processing unit, may further be configured to control the mechanical ventilator to pause or temporarily suspend ventilation of a subject during image acquisition. For example, the respiratory phases of the subject may be monitored, e.g. by obtaining the ventilation parameters and/or by obtaining monitoring parameters from one or more medical monitors, and a control signal may be generated if it is determined that the first and/or second image acquisition criterion, such as maximum inspiration or maximum expiration, is given. In this way, motion blur negatively impacting image quality may be reduced or avoided.

In an embodiment, the medical system, e.g. the data processing unit, may further be configured to control the medical device to adjust a radiation dose to be used for acquisition of the first and/or further image depending on the respective image acquisition criterion and/or the set of ventilation parameters. It is noted that, for example, the (full) expiration image as described above may be taken with a radiation dose smaller than used for the (full) inspiration image. By way of example, the radiation dose may be reduced to tenths, e.g., 5%, 10%, 20%, etc., of the radiation dose used for the (full) inspiration image.

In a second aspect, there is provided a method for controlling a medical system. Preferably, the method is computer-implemented, using computing means, such as a data processing unit, a memory, a data interface, a user interface and/or a communication interface. The method comprises the steps of:
- obtaining, by at least one data processing unit, set of ventilation parameters used for operation of a mechanical ventilator from the mechanical ventilator,
- triggering, by the data processing unit, acquisition of a first image at a medical imaging device if the set of ventilation parameters meets a first image acquisition criterion, and
- triggering, by the data processing unit, acquisition of a subsequent, further image at a medical imaging device if the set of ventilation parameters meets a second image acquisition criterion.

The method may be carried out by using the medical system of the first aspect, i.e. computational means thereof, and/or by computational means of only the mechanical ventilator or only the medical imaging device as described above, or by a combination of these computational means.

In an embodiment, the method may further comprise providing the set of ventilation parameters to a display device, providing the first and/or further image to a display device, and displaying the one or more images together with the ventilation parameters. Optionally, the ventilation parameters and the images may be assigned to each other, e.g. by time stamps, identifiers, etc., and/or stored in a memory, database, etc. In this way, changes in the lung volume in subsequent images can be checked against the ventilator settings and/or parameters.

According to an embodiment, the method may further comprise determining, e.g. calculating, estimating, etc., a lung volume, and comparing the lung volume to a previously determined lung volume. For example, if the previous lung volume of the same subject is available, this will be compared to the determined lung volume. If a derivation of the lung volumes violates a predetermined threshold, a lung volume indication signal may be generated. Optionally, a message may be displayed whether the volume has increased or decreased. Optionally, an alarm signal may be generated and output.

In an embodiment, the method may further comprise obtaining actual set of ventilation parameters from the mechanical ventilator, determining whether or not the obtained actual set of ventilation parameters complies the ventilation parameters used for acquisition of the first image and/or fulfil the first and/or second image acquisition criterion, and adjusting the set of ventilation parameters on the mechanical ventilator to comply with the ventilation parameters used for acquisition of the first image and/or to fulfil the first and/or second image acquisition. In this way, medical staff, e.g. a radiologist, can rely on ventilation conditions during the image acquisitions to be constant and that changes in the image depend only on the state of the subject.

It is noted that the above system and method allow data determined from the images, such as a lung volume, e.g. the TLC, RV, FRC, etc. as described above, and data provided by the ventilator, e.g. the ventilation parameters, by data exchange between the various system components, to be provided, displayed, stored and/or further processed together. In this way, for example, a technologist operating the medical imaging device can rely on perfect timing of image acquisition. A reading radiologist can obtain information about the ventilation parameters while assessing the image. Further, the above ventilation image or tidal volume image provides additional information, including quantitative figures. In addition, the radiologist can rely on ventilation conditions during the image acquisitions to be constant and that changes in the image depend only on the state of the subject. This implies that changes in the condition of the patient are easier to detect if the inhalation status is reproducible. Further, the above ventilation image may illustrate the local ventilation of the lungs and thus provides additional information.

It is noted that the above embodiments may be combined with each other irrespective of the aspect involved. Accordingly, the method may be combined with structural features of the system of the other aspects and, likewise, the system may be combined with features of each other, and may also be combined with features described above with regard to the method.

These and other aspects of the present invention will become apparent from and elucidated with reference to the embodiments described hereinafter.

### BRIEF DESCRIPTION OF THE DRAWINGS

Exemplary embodiments of the invention will be described in the following drawings.
Fig 1 shows in a schematic block diagram a medical system comprising a mechanical ventilator and a medical imaging device interconnected to each other, according to an embodiment.
Fig. 2 shows in a schematic block diagram a data processing unit according to an embodiment.
Fig. 3 shows in a in a schematic block diagram a data processing unit according to an embodiment.
Fig. 4 shows in a diagram different volumes associated with mechanical ventilation and/or breathing of a subject in general.
Fig. 5 shows in a flow chart a method for operating a medical system according to an embodiment.

### DETAILED DESCRIPTION OF EMBODIMENTS

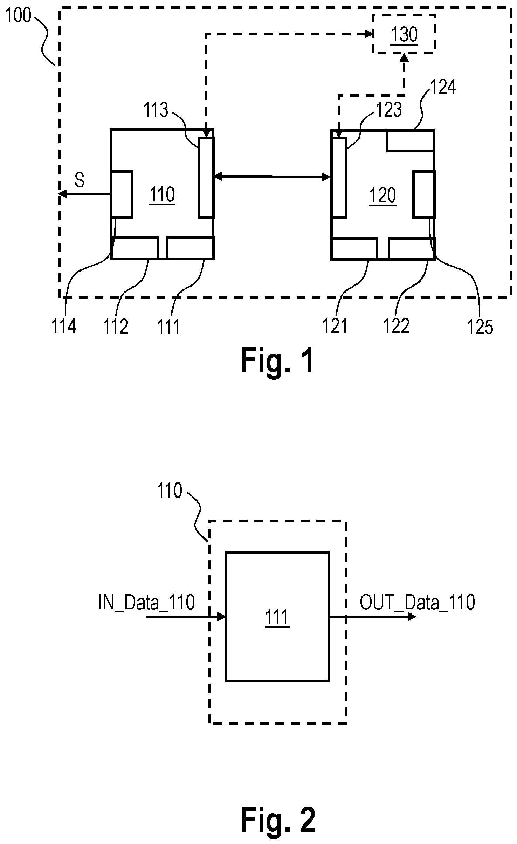

Fig. 1 shows in a schematic block diagram a medical system 100, which is particularly formed as a medical system for ventilation and imaging. It is noted that system 100 may be configured to be applied in intensive-care medicine.

System 100 comprises a mechanical ventilator 110, generally configured to provide mechanical ventilation by moving breathable air into and out of the lungs of a subject to generate breaths. Mechanical ventilator 110 comprises a controller 111, which may also be referred to as a data processing unit, an air pump 112 or other suitable means for moving air, a data and/or communication interface 113, and an ventilation interface 114 to the subject. The controller 111 is configured to control and adjust operation of ventilator 110 by controlling air pump 112 etc., and is particularly configured to operate ventilator 110 with a set of ventilation parameters to cause a desired ventilation status at the ventilated subject, such as tidal volume, minute volume in volume modalities, peak pressure (in pressure modalities), respiratory frequency, positive end expiratory pressure, inspiratory time, inspiratory flow, inspiratory-to-expiratory ratio, time of pause, trigger sensitivity, support pressure, and expiratory trigger sensitivity, etc. Optionally, mechanical ventilator 110 may comprise a monitoring system for subject-related parameters associated with the ventilation status of the ventilated subject, such as air pressure, air volume, and air flow, and/or ventilator function, such as air leakage, power failure and/or mechanical failure. Although not shown, mechanical ventilator 110 may comprise a user interface for controlling the ventilator, displaying the ventilation status and/or ventilation parameters or the like.

Further, system 100 comprises a medical imaging device 120, generally configured to acquire images of a region of interest, e.g. a chest, of the subject ventilated by mechanical ventilator 110. Preferably, medical imaging device 120 is medical ultrasound device, a radiography, X-ray, device, etc.. It comprises a controller 121, which may also be referred to as a data processing unit, a radiation source 122, e.g. an ultrasound probe, an X-ray generator and an X-ray tube, etc., a data and/or communication interface 123, a detector 124, and a user interface 125 for controlling the device and displaying images. Controller 121 is configured for image processing to provide e.g. X-ray images and further processing thereof. Further preferably, medical imaging device 120 is formed as a portable or mobile device. Further, controller 121 is configured to receive a trigger signal from system 100 and/or mechanical ventilator 110 to trigger image acquisition. The trigger signal may be generated, by system 100, i.e. controller 130, and/or mechanical ventilator 110, i.e. controller 111, based on the ventilation status information and/or ventilation parameters and/or monitoring of the ventilated subject and transmitted to medical imaging device 120, i.e. controller 121, which in turn triggers image acquisition.

It is noted that mechanical ventilator 110 and medical imaging device 120 are interconnected for data exchange, mutual control, etc. For example, data and/or communication interface 113 of mechanical ventilator 110 may be connected to the data and/or communication interface 123 of medical imaging device 120. Data to be exchanged may comprise the ventilation status information and/or ventilations parameters, an image acquired, time stamps assigned to the ventilation status and/or ventilation parameters and/or the images acquired, a trigger signal for image acquisition, feedback signals indicating that an image acquisition has been acquired, or the like.

Optionally, system 100 may further comprise a higher-level controller 130, which may also be referred to as a data processing unit, configured to control and/or monitor both the mechanical ventilator 110 and the medical imaging device 120. As indicated in Fig. 1 by dashed arrows, controller 130 may be connected via data and/or communication interface 113, 123 to the corresponding device. It is also conceivable that a higher-level control function of the system, i.e., control and/or monitoring of mechanical ventilator 110 and medical imaging device 120, is distributed among controllers 111 and 121, or that only one of them performs the higher-level control function of the overall system 100. In the latter case, the further controller 130 may be omitted.

Fig. 2 shows in a schematic block diagram controller 111, i.e. the data processing unit, of mechanical ventilator 110. It is configured to receive, e.g. via its data and/or communication interface 113, input data IN_Data_110, which may comprise one or more of a control command or signal for setting the above-mentioned ventilation status and/or ventilation parameters, a control command or signal for temporarily pausing ventilation during an image acquisition, a control command or signal for outputting the current ventilation status and/or ventilation parameters, etc. Further, controller 111 is configured to output, e.g. via its data and/or communication interface 113, output data OUT_data 110, which may comprise one or more of currently used and/or intended ventilation status and/or ventilation parameters, a control command or signal to trigger an image acquisition, a timestamp associated with the ventilation status and/or ventilation parameters, etc. It is noted that the data processing underlying the output data OUT_data_110 may also be at least partially outsourced to another data processing unit, such as controller 130 and/or controller 121.

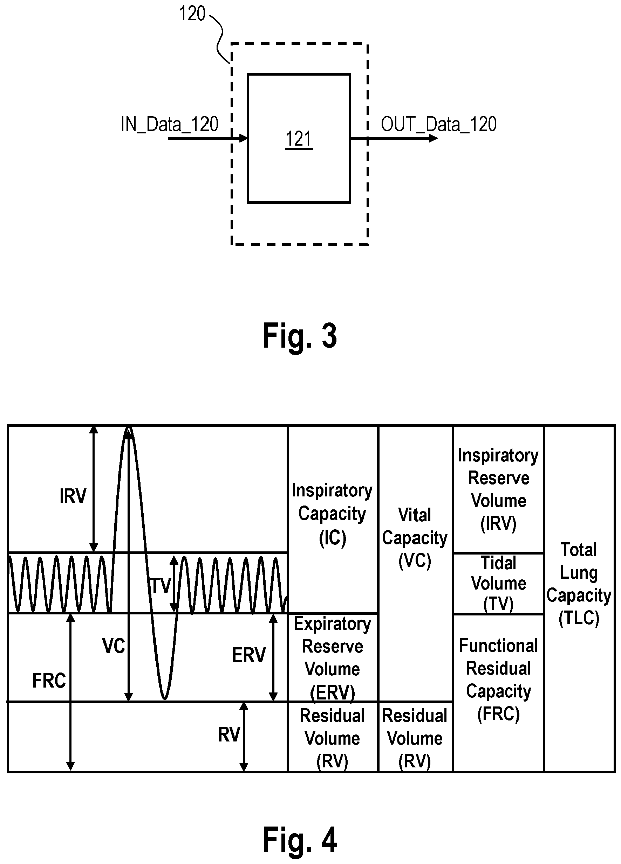

Fig. 3 shows in a schematic block diagram controller 121, i.e. the data processing unit, of medical imaging device 120. It is configured to receive, e.g. via its data and/or communication interface 123, input data IN_Data_120, which may comprise one or more of a control command or signal to trigger an image acquisition, a control command or signal to generate a specific image, such as an inspiration image, expiration image, tidal volume image, respiratory image, etc. Further, controller 121 is configured to process image data to generate the specific images through image processing techniques such as segmentation of captured images, subtraction of captured images from each other. It is noted that some or all of this image data processing may also be at least partially outsourced to another data processing unit, such as controller 130. Furthermore, controller 121 is configured to output, e.g. via its data and/or communication interface 123, output data OUT_data_120, which may comprise one or more of an image and/or image data, comprising e.g. DICOM data etc., acquired at moment triggered by the control command or signal to trigger image acquisition, a specified image, such as an (full) inspiration image, (full) expiration image, tidal volume image, respiratory image, etc., a timestamp associated with the image output, etc.

For example, the control command or signal for triggering image acquisition may be based on a first image criterion and/or a second image acquisition criterion. These image acquisition criteria may comprise maximum inspiration of the ventilated subject. Accordingly, the respective image acquired at maximum inspiration may be referred to as "inspiration image". Likewise, the image acquisition criteria may comprise maximum expiration of the to- be-ventilated or ventilated subject. Accordingly, the respective image acquired at maximum expiration may be referred to as "expiration image". Controller 121 may be configured to acquire, process and provide the respective inspiration and/or expiration image, e.g. via the data and/or communication interface 123.

By way of example, controller 121 may be configured to determine a lung volume from the image at maximum inspiration, i.e. the full inspiration image, which measured volume may be referred to as "total lung capacity (TLC)". Further, for example, a lung volume may be determined from the image at maximum full expiration, i.e. the full expiration image, which measured volume may be referred to as "residual volume (RV)". Further, for example, a lung volume may be determined from the image at maximum expiration, i.e. the expiration image, which measured volume may be referred to as "functional residual capacity (FRC)". For example, TLC and/or RV may be determined by generating and/or using a respective 2D X-ray image of the subject's chest. The image may be segmented to identify lung structures to provide segmented image data separated from un-segmented areas. Then, spatial lung volume information may be extracted from the image data using the segmented image data derived from the image data. A method to identify such lung structures is described in US 2016/0210739 A1, the content of which is incorporated herein in its entirety. Thereby, in a respective area of the lungs structure in the X-ray image, the intensity of the image at that area is related to the amount of air present in the lung structure at that area on the image. Therefore, for each respective area over the lung structure, a summation of the pixel intensity values corresponding to that area can allow an approximation of the lungs volume to be found for that area. According to an exemplary embodiment, image data of the first and/or further image may also comprise pixel area information, in other words, the pixel pitch of the detector in e.g. mm², cm², etc. Therefore, after segmentation, the area of each lung in mm² can be calculated. The darkness or brightness of the lungs is a measure of a transition passage filled with air, so it can be re-mapped to the area of the lung in millimeters or centimeters squared. Integrating the pixel area over the lung structure leads to a figure, which is closely correlated to the volume of the lungs. Controller 121 may be configured to determine and provide the respective RV and/or TLC and/or FRC, e.g. via the data and/or communication interface 123.

Further, by way of example, controller 121 may further be configured to determine vital capacity (VC) information from one of the first and further image assigned to a residual volume, RV, and the other one of the first and further image assigned to a total lung capacity, TLC, by determining, e.g. calculating, a difference between the RV and the TLC. As described above, TLC and RV may be determined from the respective images acquired at maximum full inspiration and maximum full expiration. By forming the difference of TLC and RV, the vital capacity may be determined. Similarly, the tidal volume (TV) can be determined from the maximum inspiration image and maximum expiration image.

Controller 121 may be configured to determine and provide the vital capacity and/or the tidal volume information, e.g. via the data and/or communication interface 123.

Further, in an exemplary embodiment, controller 121 may be configured to generate a respiratory image by subtracting the image acquired at maximum (full) expiration and the image acquired at maximum (full) inspiration from each other. The respiratory image shows regional ventilation or spatial tidal volume. Controller 121 may be configured to determine and provide the respiratory image, e.g. via the data and/or communication interface 123.

With reference to Fig. 4, the volumes mentioned above are now described. Fig. 4 shows in a diagram different volumes associated with mechanical ventilation and/or breathing in general. Thereby, as used herein, RV corresponds to or correlates with maximum full expiration, FRC corresponds to or correlates with maximum expiration, FRC+TV maximum inspiration, and TLC corresponds to or correlates with maximum full inspiration.

Note that RV and/or TLC may not be fully achieved by mechanical ventilation, but can at least be approximated or nearly achieved.

Referring again to Fig. 3, in a further exemplary embodiment, controller 121 may be configured to generate another image, which may be referred to as a "ventilation image", by displaying the one of the first and second image acquired at maximum (full) expiration based on a histogram of the other one of the first and second image acquired at maximum (full) inspiration. In other words, the (full) expiration image may be displayed based on the histogram of the (full) inspiration image. Controller 121, which may carry out suitable image processing techniques, the image(s) may be rendered as appropriate. Thereby, the (full) expiration image is brighter than the (full) inspiration image at positions, areas, sections, etc., where there is less air in the lungs. The positions, areas, sections, etc. where there is less air in the lungs indicates good ventilation. In contrast, darker positions, areas, etc. within the (full) expiration image indicate little or less air exchange. For example, controller 121 may be further configured to divide, e.g. to segment etc., the ventilation image into sections of different brightness by means of image processing. Optionally, sections of different brightness may be highlighted by using different colors, for example green color for sections indicating less air in the lungs and red color for sections indicating little or less air exchange of the lungs. Controller 121 may be configured to determine and provide the ventilation image, e.g. via the data and/or communication interface 123.

Further, by way of example, controller 121 may further be configured to include one or more of the ventilation status information and a set of ventilation parameters used to operate mechanical ventilator 110, used to acquire the respective image(s) acquired into image data assigned to the image(s) acquired. Controller 121 may be configured to determine and provide the combined ventilation status information and/or ventilation parameters and image data together, e.g. via the data and/or communication interface 123.

Referring to Fig. 1, which shows system 100 in a schematic block diagram, the function of system 100 and the interaction of the individual system components with each other will now be explained.

As described above, the ventilation status information, i.e. the current ventilation status of the ventilated subject, is obtained from mechanical ventilator 110 by one or more of the above controllers 111, 121, 130. Alternatively or additionally, a measurement of the breathing activity of the or ventilated subject may be performed. Further alternatively or additionally, a number of images, e.g. a number of X-ray frames, may be acquired by using medical imaging device 120, for example, with 5, 10, 15, 20 fps, and the lung volume may be determined and/or monitored.

Based on the obtained knowledge on the current respiratory phase of the subject, one or more of the above controllers 111, 121, 130, may trigger image acquisition of a first image if the ventilation status information , i.e. the current ventilation status, and/or current respiratory phase of the ventilated subject meets a first image acquisition criterion. This criterion may be at least one of that explained above, namely maximum inspiration, maximum full inspiration, maximum expiration, maximum full expiration, etc.

Further, one or more of the above controllers 111, 121, 130 may trigger image acquisition of at least one subsequent, further image if the current ventilation status and/or current respiratory phase of the ventilated subject meets a second image acquisition criterion.

It is noted that the first and second image acquisition criterion may be the same or deviate from each other at most within a tolerance window, e.g. ±1%, ±2%, ±3, ... ±10%, or the like. In this case, the first image acquisition criterion may be stored in computer-readable memory and may be obtained and/or evaluated to determine the second image acquisition criterion therefrom, which is the same as the first image acquisition criterion. Further, in this case, one or more of the above controllers 111, 121, 130 may further be configured to control adjustment of e.g. the ventilation status cause, e.g. by adjusting a set of ventilation parameters, on mechanical ventilator 110 to be operated if it is determined that the ventilation status and/or one or more of the ventilation parameters have changed compared to the acquisition of the first image. In another case, the first and/or second image acquisition criterion may differ from each other. For example, one image acquisition criterion may be associated with maximum (full) inspiration while the other image acquisition criterion may be associated with maximum (full) expiration. In this case, one or more of the above controllers 111, 121, 130 may further be configured to obtain, evaluate and, if necessary, adjust the ventilation status, e.g. by adjusting the ventilation parameters of mechanical ventilator 110 according to the image acquisition criterion to be met.

Furthermore, the acquired image, or any of the generated images as explained above, may be provided, by medical imaging device 120 or the respective one of controllers 111, 121, 130 to medical system 100 and/or mechanical ventilator 110 for e.g. displaying, storing, etc.

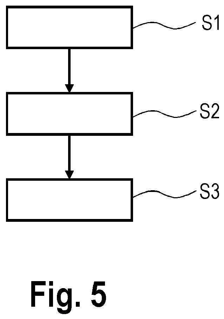

Fig. 5 shows in a flow chart a method for controlling medical system 100.

In a step S1, the ventilation status information associated with the ventilation status of the subject caused by mechanical ventilator 110 is obtained in a way as described above. In a step S2, acquisition of a first image at using medical imaging device 120 is triggered if the ventilation status information or the ventilation status itself and/or the respiratory phase of the ventilated subject meets the first image acquisition criterion as explained above. In a step S3, acquisition of a subsequent, further image at medical imaging device 120 if the ventilation status and/or the respiratory phase of the subject meets a second image acquisition criterion as explained above.

In another exemplary embodiment, a computer program or computer program element is provided that is characterized by being configured to execute the method steps of the method according to one of the preceding embodiments, on an appropriate system, such as system 100.

The computer program element might therefore be stored on a data processing unit, which might also be part of an embodiment. This data processing unit may be configured to perform or induce performing of the steps of the method described above. Moreover, it may be configured to operate the components of the above described device and/or system. The computing unit can be configured to operate automatically and/or to execute the orders of a user. A computer program may be loaded into a working memory of a data processor. The data processor may thus be equipped to carry out the method according to one of the preceding embodiments.

Further, the computer program element might be able to provide all necessary steps to fulfill the procedure of an exemplary embodiment of the method as described above.

According to a further exemplary embodiment of the present invention, a computer readable medium, such as a CD-ROM, USB stick or the like, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It is noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and the foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: medical system
- 110: mechanical ventilator
- 111: controller/data processing unit
- 112: air pump
- 113: data and/or communication interface
- 120: medical imaging device
- 121: controller/data processing unit
- 122: radiation source
- 123: data and/or communication interface
- 124: detector
- 125: user interface
- 130: controller/data processing unit

## Claims

1. A medical system (100), comprising:
at least one data processing unit (111, 121, 130),
a mechanical ventilator (110), configured to be operated to cause a ventilation status at a to-be-ventilated subject,
a medical imaging device (120,
wherein the data processing unit (111, 121, 130) is configured to obtain, from the mechanical ventilator (110), a ventilation status information associated with the ventilation status of the to-be-ventilated subject,
wherein the data processing unit (111, 121, 130) is further configured to trigger acquisition of a first image if the ventilation status meets a first image acquisition criterion, and
wherein the data processing unit (111, 121, 130) is further configured to trigger acquisition of at least one subsequent, further image if the ventilation status meets a second image acquisition criterion.

2. The medical system of claim 1, wherein the first and second image acquisition criterion are the same or deviate at most within a tolerance window.

3. The medical system of claim 2, wherein the data processing unit (111, 121, 130) is further configured to control adjustment of a set of ventilation parameters on the mechanical ventilator (110) based on which the ventilation status is caused if the data processing unit (111, 121, 130) determines that the ventilation status has changed compared to the acquisition of the first image.

4. The medical system of claim 1, wherein the first and second image acquisition criterion differ from each other.

5. The medical system of any one of the preceding claims, wherein at least one of the first and second image acquisition criterion is maximum inspiration of a ventilated subject.

6. The medical system of any one of the preceding claims, wherein at least one of the first and second image acquisition criterion is minimum inspiration or maximum expiration of a ventilated subject.

7. The medical system of claim 5 and 6, wherein the data processing unit (111, 121, 130) is further configured to generate a ventilation image by displaying the one of the first and second image acquired at minimum inspiration or maximum expiration based on a histogram of the other one of the first and second image acquired at maximum inspiration.

8. The medical system of claim 7, wherein the data processing unit (111, 121, 130) is further configured to determine one or more positions where the image acquired at minimum inspiration or maximum expiration has higher brightness than indicated by the histogram of the image acquired at maximum inspiration.

9. The medical system of claim 7 or 8, wherein the data processing unit (111, 121, 130) further configured to control displaying the ventilation image at the mechanical ventilator (110).

10. The medical system of claim 5 and 6, wherein the data processing unit (111, 121, 130) is further configured to generate a respiratory image by subtracting the image acquired at minimum inspiration or maximum expiration and the image acquired at maximum inspiration from each other.

11. The medical system of any one of the preceding claims, wherein the data processing unit (111, 121, 130) is further configured to determine tidal volume information from the one of the first and further image assigned to a residual volume, RV, and the other one of the first and further image assigned to a total lung capacity, TLC, by determining a difference between the RV and the TLC.

12. The medical system of any one of the preceding claims, wherein the data processing unit (111, 121, 130) is further configured to include the ventilation status information used to acquire the first and/or further image into image data assigned to the first and/or further image.

13. The medical system of any one of the preceding claims, wherein the data processing unit (111, 121, 130) is further configured to control the mechanical ventilator (110) to pause or suspend ventilation of a subject during image acquisition.

14. The medical system of any one of the preceding claims, wherein the data processing unit (111, 121, 130) is further configured to control the medical device to adjust a radiation dose to be used for acquisition of the first and/or further image depending on the respective image acquisition criterion and/or the ventilation status information.

15. A method for controlling a medical system, comprising:
- obtaining, by at least one data processing unit (111, 121, 130), a ventilation status of to-be-ventilated subject caused by operation of a mechanical ventilator (110) from the mechanical ventilator (110),
- triggering, by the data processing unit (111, 121, 130), acquisition of a first image at a medical imaging device (120) if the ventilation status meets a first image acquisition criterion, and
- triggering, by the data processing unit (111, 121, 130), acquisition of a subsequent, further image at a medical imaging device (120) if the ventilation status meets a second image acquisition criterion.
